# EUROPÄISCHE PATENTSCHRIFT

(11) **EP 2 389 879 B1**
(45) Veröffentlichungstag und Bekanntmachung des Hinweises auf die Patenterteilung: **21.02.2018**
(21) Anmeldenummer: 11165060.2
(22) Anmeldetag: 06.05.2011
(51) Int. Cl.: A61B 17/16, A61B 17/32, A61B 17/3207

(54) **Medizinisches Instrument mit abnehmbarem Griff**
Medical instrument with removable handle
Instrument médical doté d'une poignée amovible

(30) Priorität: 10.05.2010 DE 102010020927
(43) Veröffentlichungstag der Anmeldung: 30.11.2011
(73) Patentinhaber: Karl Storz SE & Co. KG, 78532 Tuttlingen (DE)
(72) Erfinder: Simmen, Markus, 8603 Schwarzenbach (CH); Spycher, Raphael, 8400 Winterthur (CH); Vogel, Urs, 8200 Schaffhausen (CH)
(74) Vertreter: Witte, Weller & Partner Patentanwälte mbB

(56) Entgegenhaltungen:
- WO-A1-97/24072
- WO-A1-03/079911
- US-A1- 2002 077 530
- US-A1- 2007 034 394
- US-A1- 2007 244 353
- US-A1- 2009 163 935

## Beschreibung

Die vorliegende Erfindung betrifft ein medizinisches Instrument zum Abtragen von Gewebe, in Form eines eines Shavers, Bohrers, oder Morcellators, mit einem in Rotation versetzbaren Werkzeug und einem motorischen Antrieb zum Antreiben des Werkzeuges, und mit einem langgestreckten Körper, wobei am Körper Anschlüsse vorhanden sind, von denen ein Anschluss ein Stromanschluss ist, und einem seitlich von diesem langgestreckten Körper abstehenden Handgriff, der abnehmbar an dem langgestreckten Körper befestigt ist, so dass das medizinische Instrument bei angebrachtem Handgriff über diesen ergreifbar ist.

Aus der WO 03/079911 A1 ist ein medizinisches Instrument zum Abtragen von Gewebe in Form eines Shavers, Bohrers oder Morcellators bekannt. Dieses Instrument weist ein in Rotation versetzbares Werkzeug und einen motorischen Antrieb zum Antreiben des Werkzeuges auf. Ferner ist ein langerstreckter Körper vorhanden, von dem seitlich ein Handgriff vorsteht. Dieser Handgriff ist mittels Schrauben an dem Körper befestigbar und bei Lösen dieser Schrauben vom Körper abnehmbar.

Aus der US 2007/034394 A1, die sich auf das Gebiet der Handwerkzeuge bezieht, ist eine Handbohrmaschine bekannt, mit einem Gehäuse und einem Handgriff, der als Pistolengriff angeordnet ist, wobei der Handgriff ferner in einer gelösten Position vom Gehäuse und einer axial mit einer Längsachse des Gehäuses ausgerichteten Position angeordnet werden kann. Der Handgriff trägt einen Energiespeicher. Auch in der gelösten Position bleibt der Handgriff über ein Kabel mit dem Gehäuse der Handbohrmaschine verbunden.

Die Erfindung betrifft ferner einen Handgriff zur Verwendung mit einem medizinischen Instrument der vorstehend genannten Art.

Medizinische Instrumente, insbesondere Shaver mit einem langgestreckten Körper und einem seitlich von diesem langgestreckten Körper abstehenden Handgriff, sind bekannt und werden z.B. von der Anmelderin unter der Bezeichnung DrillCut-X vertrieben. Bei diesem Produkt handelt es sich um einen so genannten Shaver zur Anwendung in der HNO-Medizin. Solche Instrumente werden in der minimalinvasiven Chirurgie zum Abtragen von Gewebe im menschlichen oder tierischen Körper verwendet. Diese Instrumente weisen üblicherweise einen langgestreckten Körper auf, der an seinem distalen Ende ein in Rotation versetzbares Werkzeug aufweist. Der Shaver-Werkzeugschaft weist üblicherweise einen feststehenden Außenschaft mit einem meist abgerundeten Ende und einem darin rotierenden Innenschaft auf. Der Außenschaft weist im Bereich seines distalen Endes zumindest ein Fenster mit zumindest einer Schneide auf. Der Innenschaft weist dabei an seinem distalen Ende im Bereich des zumindest einen Fensters des Außenschafts zumindest ein Schneidelement auf, das z.B. ebenfalls in Form eines Fensters mit einer Schneide ausgebildet sein kann. Im Betrieb rotiert nun der Innenschaft innerhalb des Außenschafts und Gewebe, das in das Fenster vom Außenschaft eintritt, wird dann zwischen der Schneide des Fensters und den rotierenden Schneidelementen des Innenschafts abgetragen und durch den Innenschaft, z.B. durch Anlegen eines Vakuums, abgesaugt. Der langgestreckte Körper des Shavers weist üblicherweise den Motor zum Antrieb des Innenschafts und damit des rotierenden Schneidwerkzeugs auf. Der seitlich abstehende Griff ermöglicht es dem Operateur, das medizinische Instrument zu greifen und für die Operation zu manipulieren.

Es sind aber auch Shaver bekannt, bei denen der Motor im Handgriff angeordnet ist.

Es hat sich nun im praktischen Betrieb allerdings gezeigt, dass ein einzelner feststehender Handgriff im Hinblick auf verschiedene Einsatzpositionen des Shavers ungünstig ist. Es hat sich ferner gezeigt, dass verschiedene Anwender verschiedene Handhaltungen beim Benutzen des Shavers bevorzugen, wobei manche sogar vollständig auf den Griff verzichten und den langgestreckten Shaver-Körper z.B. ähnlich einem Stift zu halten pflegen.

Die vorliegende Erfindung ist nicht auf Shaver beschränkt, sondern das erfindungsgemäße Instrument kann bspw. auch ein chirurgischer Bohrer oder auch ein Morcellator sein. Der Begriff "Werkzeug" ist entsprechend im Fall eines Bohrers als Bohrwerkzeug, im Fall eines Shavers oder Morcellators, als Schneidwerkzeug, und bei noch anderen Instrumenten zum Abtragen von Gewebe bspw. auch als Fräs- oder Raspelwerkzeug zu verstehen.

Es ist eine Aufgabe der Erfindung, ein medizinisches Instrument zu beschreiben, das gegenüber den bekannten Instrumenten eine deutlich höhere Flexibilität hinsichtlich der Ergonomie aufweist und auf eine Vielzahl von Haltepositionen angepasst werden kann.

Erfindungsgemäß wird die Aufgabe hinsichtlich des eingangs genannten medizinischen Instruments dadurch gelöst, dass das medizinische Instrument bei abgenommenem Handgriff über den langgestreckten Körper in Art eines Stabes haltbar ist, dass der motorische Antrieb in Form eines Elektromotors ausgebildet ist, der im langgestreckten Körper angeordnet ist, und dass der Handgriff so ausgebildet ist, dass er sowohl in distaler Richtung als auch in proximaler Richtung abgewinkelt an dem langgestreckten Körper anbringbar ist.

Auf diese Weise wird ein Instrument geschaffen, bei dem der seitlich abstehende Handgriff abnehmbar oder anbaubar ist, so dass je nach bevorzugter Handhaltung das Instrument über den seitlich abstehenden Griff oder z.B. in der Art eines Stabs gehalten werden kann. Ferner können durch den Handgriff bereits bestehende medizinische Instrumente mit langgestrecktem Körper mit einem seitlich abstehenden Handgriff ausgestattet werden.

Der Begriff "Schienen", wie er in der vorliegenden Anmeldung verwendet wird und wie sie im oberen Bereich der Ausnehmung des erfindungsgemäßen Handgriffs vorgesehen sind, beschreibt jegliche Konstruktion, die in der Lage ist, in korrespondierende Nuten einzugreifen und eine geführte Halterung des medizinischen Instruments zu erreichen. Diese Schienen können eine jegliche Länge aufweisen, wobei sie in einem Extremfall auf einen Zapfen reduziert sein können und im anderen Extremfall sich über die gesamte Länge der Ausnehmung erstrecken können. Es ist auch möglich, dass die Schienen sich aus mehreren kleineren Einzelschienen zusammensetzen.

In einer Ausgestaltung der Erfindung kann der Handgriff in verschiedenen Positionen entlang der Längsachse an dem Körper festgelegt werden. Insbesondere kann der Handgriff stufenlos in verschiedenen Positionen entlang der Längsachse des Körpers festgelegt werden.

Diese Maßnahme erhöht die Flexibilität der Ergonomie des erfindungsgemäßen medizinischen Instruments weiter, da dadurch ein Operateur Positionen des Handgriffes noch stärker auf die operativen Gegebenheiten sowie seine persönlichen Vorlieben einstellen kann. Ferner kann dadurch das Instrument auch auf einfache Weise von verschiedenen Operateuren mit verschiedenen Vorlieben oder auch z.B. verschieden großen Händen verwendet werden.

In einer Ausgestaltung der oben genannten Maßnahme weist der Handgriff eine Verriegelung auf, mit der er in einer Position entlang der Längsachse des Körpers verriegelt werden kann.

Auch wenn es möglich ist, die Festlegung des Handgriffs in verschiedenen Positionen entlang der Längsachse des Körpers auf andere Weisen zu erreichen als mit einer Verriegelung, ist die Bereitstellung einer Verriegelung vorteilhaft, da bei geöffneter Verriegelung der Handgriff auf einfache Weise von dem medizinischen Gerät entfernt werden kann und bei geschlossener Verriegelung der Handgriff gegen ein Verrutschen aus der festgelegten Position sicher geschützt ist. Diese Ausgestaltung trägt auch zur Betriebssicherheit des Instruments bei, die wichtig ist, da es sich bei dem Instrument um ein solches handelt, mit dem zügig Gewebe abgetragen wird und das daher stets unter Kontrolle sein muss. Auch muss der Handgriff bei etwaigen Vibrationen, die durch den motorischen Antrieb verursacht werden können, fest am Instrument sitzen.

Die Verriegelung kann hierbei je nach dem, ob diese in Stufen oder stufenlos erfolgt, auf alle dem Fachmann bekannte Weisen erfolgen. Möglichkeiten hierzu sind federbelastete Stift- oder Kugelverriegelungen, die in Sacklöcher eingreifen, Rastnasen oder Verriegelungen, bei denen z.B. die Schienen in irgendeiner Weise gegen das Instrument oder das Instrument gegen die Schienen gedrückt wird.

Der Handgriff kann zur Anbringung an den Körper des Instruments eine voll- oder teilumfängliche geschlossene Ausnehmung aufweisen, in der der Körper aufgenommen ist.

In einer bevorzugten Ausgestaltung weist der Handgriff des medizinischen Instruments eine Ausnehmung zur Aufnahme des Körpers auf, wobei im oberen Bereich der Ausnehmung zumindest zwei im Wesentlichen parallele, einander gegenüberliegende Schienen vorgesehen sind, die in dem Zustand, in dem der Handgriff mit dem medizinischen Gerät verbunden ist, in entsprechende, an dem Körper angeordnete Nuten eingreifen.

Diese Ausgestaltung hat den Vorteil, dass sie baulich einfach ist, eine stufenlose Veränderung der Position des Handgriffs ermöglicht und gleichzeitig jedoch eine sichere und feste Führung des Körpers des medizinischen Instruments an dem Handgriff sicherstellt.

In einer Ausgestaltung der oben genannten Maßnahme weist der Handgriff ferner zumindest einen verstellbaren Finger auf, der aus einer Stellung, in der eine Spitze des Fingers in einer Innenfläche der Ausnehmung versenkt ist, gegen den Körper verspannbar ist, um den Handgriff am Körper zu verriegeln, bzw. aus der der Finger in Richtung zum Innenraum der Ausnehmung hin bewegbar ist.

In dieser Ausführungsform wird eine Verriegelung wie folgt erreicht. In einem ersten Schritt wird das medizinische Instrument in den Handgriff eingeschoben, wobei sich der Finger in der versenkten Stellung befindet und wobei die Schienen des Handgriffs in an dem Instrument vorgesehenen Nuten eingreifen. In einem zweiten Schritt wird nun der Finger gegen den Körper des Instruments gedrückt, so dass nun das Instrument im Wesentlichen von unten gegen die Schienen gedrückt wird. Es hat sich gezeigt, dass diese Konstruktion zum einen eine einfache Ver- und Entriegelung des Handgriffs ermöglicht und zum anderen zu einer besonders sicheren und festen Verriegelung führt.

In einer weiteren Ausgestaltung der Erfindung steht der Handgriff in einem Winkel von dem Körper ab, der ungleich 90° ist bzw. ist ein Winkel zwischen der Längsachse der Ausnehmung und der Längsachse des Handgriffs ungleich 90°.

Es hat sich gezeigt, dass Handgriffe, die in einem anderen Winkel als um 90° von dem langgestreckten Körper abstehen, vom Benutzer häufig als ergonomisch angenehmer empfunden werden.

Der Handgriff ist so ausgebildet, dass er sowohl in distaler als auch in proximaler Richtung abgewinkelt an dem Körper angebracht werden kann.

Dies bedeutet, dass der Handgriff bezogen auf eine Linie, die im rechten Winkel zur Längsachse des langgestreckten Körpers steht, entweder nach vorne oder nach hinten ausgelenkt ist. Dadurch, dass der Handgriff so ausgelegt ist, dass er in beide Richtungen ausgelenkt an dem Körper angebracht werden kann, wird die Flexibilität der Ergonomie weiter erhöht und das medizinische Instrument kann z.B. an einen eher nach oben gerichteten Arbeitswinkel oder einen eher nach unten gerichteten Arbeitswinkel angepasst werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand ausgewählter Ausführungsbeispiele im Zusammenhang mit den Zeichnungen näher beschrieben und erläutert. Es zeigen:
Fig. 1 ein medizinisches Instrument in Form eines Shavers in Seitenansicht;
Fig. 2 eine erste Konfiguration des medizinischen Instruments von Fig. 1;
Fig. 3 eine zweite Konfiguration des medizinischen Instruments von Fig. 1;
Fig. 4 eine dritte Konfiguration des medizinischen Instruments von Fig. 1;
Fig. 5 eine vierte Konfiguration des medizinischen Instruments von Fig. 1;
Fig. 6 eine Stirnansicht eines Handgriffs zur Verwendung mit einem medizinischen Instrument; und
Fig. 7 eine Seitenansicht des Handgriffs von Fig. 6 im Schnitt.

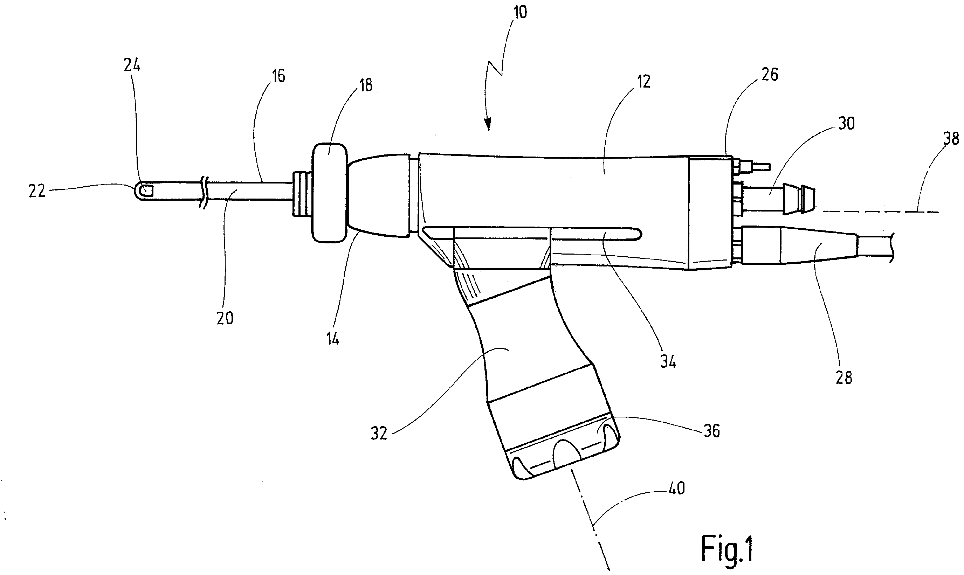

In Fig. 1 ist ein medizinisches Instrument in seiner Gesamtheit mit der Bezugsziffer 10 bezeichnet.

Bei dem medizinischen Instrument 10 handelt es sich ohne Beschränkung der Allgemeinheit um einen Shaver. Dieser Shaver weist einen langgestreckten Körper 12 auf, wobei an dessen distalem Ende 14 ein Shaver-Werkzeugschaft 16 angeordnet ist.

Der Shaver-Werkzeugschaft 16 weist eine Kupplung 18 zur Ankopplung an den Körper 12 des Shavers sowie einen Außenschaft 20 auf, der an seinem distalen Ende in einem abgerundeten Ende 22 endet. Ferner ist im Bereich des distalen Ende des Außenschafts 20 ein Fenster 24 angebracht, welches mit Schneiden ausgestattet ist und einen Teil des Schneidewerkzeugs des Shavers bildet.

Am proximalen Ende 26 des Körpers 12 sind Anschlüsse 28 und 30 vorgesehen. Anschluss 28 ist ein Stromanschluss, der einen in dem Körper 12 angeordneten und hier nicht dargestellten Elektromotor mit Strom versorgt. Dieser Elektromotor versetzt einen innerhalb des Außenschafts 20 des Werkzeugschafts 16 angeordneten und hier nicht dargestellten Innenschaft in Rotation. Die Rotation des Innenschafts gegenüber dem feststehenden Außenschaft 20 des Werkzeugs führt, da der Innenschaft im Bereicht des Fensters 24 mit Schneiden versehen ist, dann zu der gewünschten Schneidwirkung.

Der Anschluss 30 am proximalen Ende 26 des Körpers 12 dient dazu, die Innenseite des Werkzeugschafts 16 mit einem Vakuum zu beaufschlagen, so dass durch die Schneidwirkung abgetragenen Gewebestücke durch den langgestreckten Körper 12 aus dem Instrument abgesaugt werden.

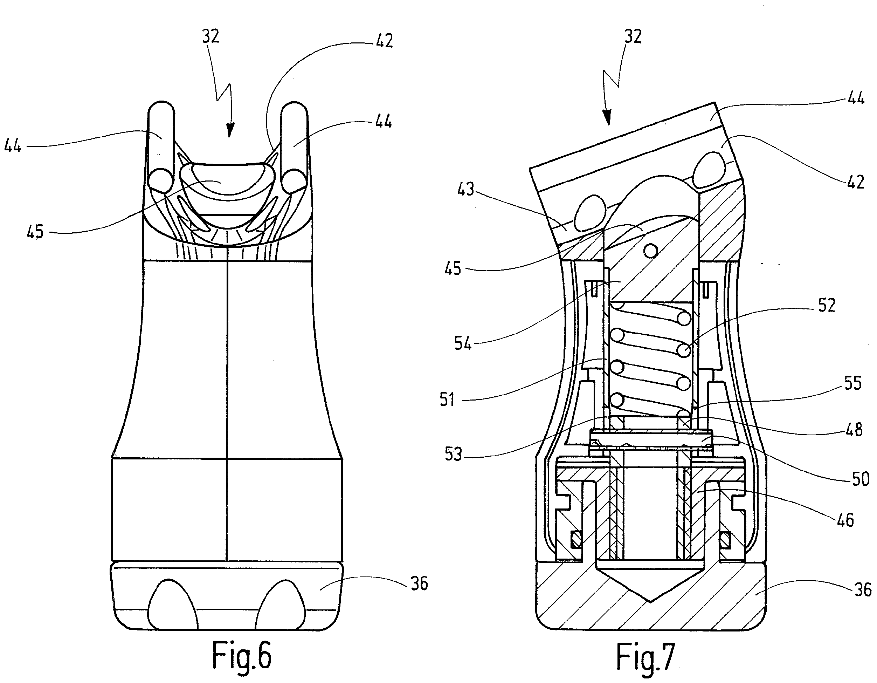

An der Unterseite des langgestreckten Körpers 12 ist ein Handgriff 32 angeordnet. Die Anordnung des Handgriffs 32 erfolgt hierbei dadurch, dass an dem Handgriff 32 angeordnete Schienen in eine Nut 34 im Körper 12 des medizinischen Instruments 10 eingreifen. Der Handgriff 32 weist an seinem unteren Ende einen Drehknopf 36 auf, der dazu dient, den Handgriff 32 an dem Körper 12 des medizinischen Instruments 10 zu verriegeln. Die Funktionsweise des Handgriffs 32 wird näher anhand eines einzelnen Handgriffs in den Figuren 6 und 7 beschrieben.

In den dargestellten Konfigurationen ist der Handgriff 32 in Richtung des proximalen Endes 26 des Körpers 12 abgewinkelt, das heißt der Winkel zwischen der Längsachse des Körpers 12, die hier durch eine gestrichelte Linie 38 dargestellt ist, und der Längsachse des Handgriffs 32, die durch eine strichgepunktete Linie 40 dargestellt ist, ist ungleich 90°.

Aufgrund der Ausbildung der Verbindung zwischen dem Handgriff 32 und dem Körper 12 in Form von Schienen und Nuten 34 ist der Handgriff 32 stufenlos in verschiedenen Positionen entlang der Längsachse des Körpers 12 festlegbar. Dadurch, dass die Nuten 34 in dem Körper 12 ferner in Richtung des distalen Endes 14 des Körpers 12 offen sind, kann der Handgriff 32 auch vollständig von dem Körper 12 abgezogen werden, so dass der Shaver dann z.B. in der Art eines Stabes gehalten werden kann. Es besteht ferner die Möglichkeit, den Handgriff 32 um 180° zu drehen und in der umgekehrten Orientierung wieder an dem Körper 12 festzulegen. In diesem Fall wäre der Handgriff 32 in Richtung des distalen Endes 14 des Körpers 12 abgewinkelt.

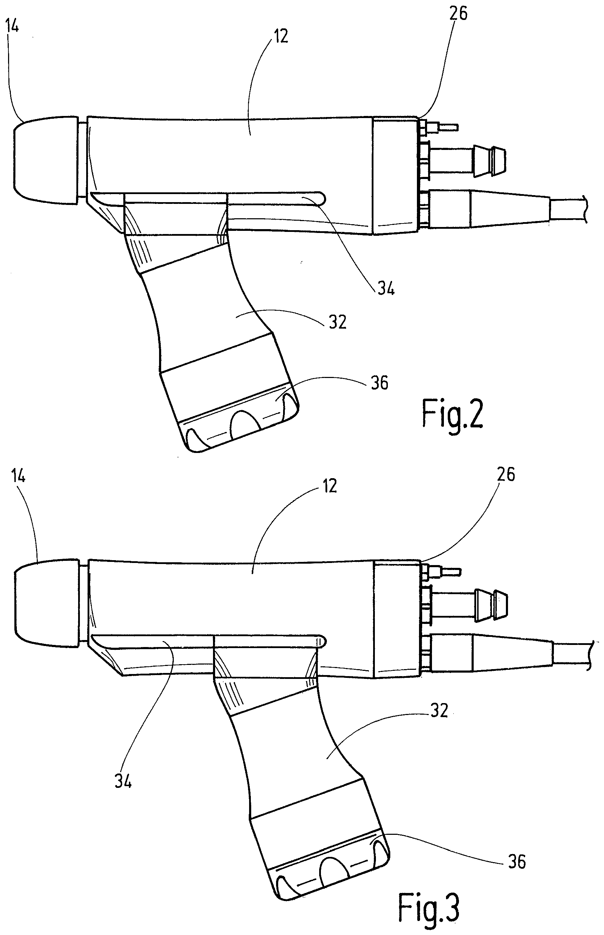

Verschiedene Konfigurationen, die auf diese Weise erreicht werden können, sind in den Figuren 2 bis 5 dargestellt, wobei aus Gründen der Einfachheit der Shaver-Werkzeugschaft 16 in den Figuren 2 bis 5 nicht dargestellt ist.

Die in Fig. 2 dargestellte Konfiguration entspricht der von Fig. 1.

Die in Fig. 3 dargestellte Konfiguration unterscheidet sich dadurch von der in Fig. 2 dargestellten Konfiguration, dass der Handgriff gegenüber Fig. 2 in Richtung des proximalen Endes 26 des Körpers 12 verschoben wurde. Um den Handgriff in diese Stellung zu bringen, muss lediglich durch Drehen des Drehknopfes 36 die Verriegelung gelöst werden und dann der Handgriff 22 entlang der Nut 34 in Richtung des proximalen Endes 26 des Körpers 12 verschoben werden. Hat der Handgriff 32 die gewünschte Stellung erreicht, wird der Drehknopf 36 in umgekehrte Richtung gedreht und der Handgriff 32 erneut an dem Körper 12 verriegelt.

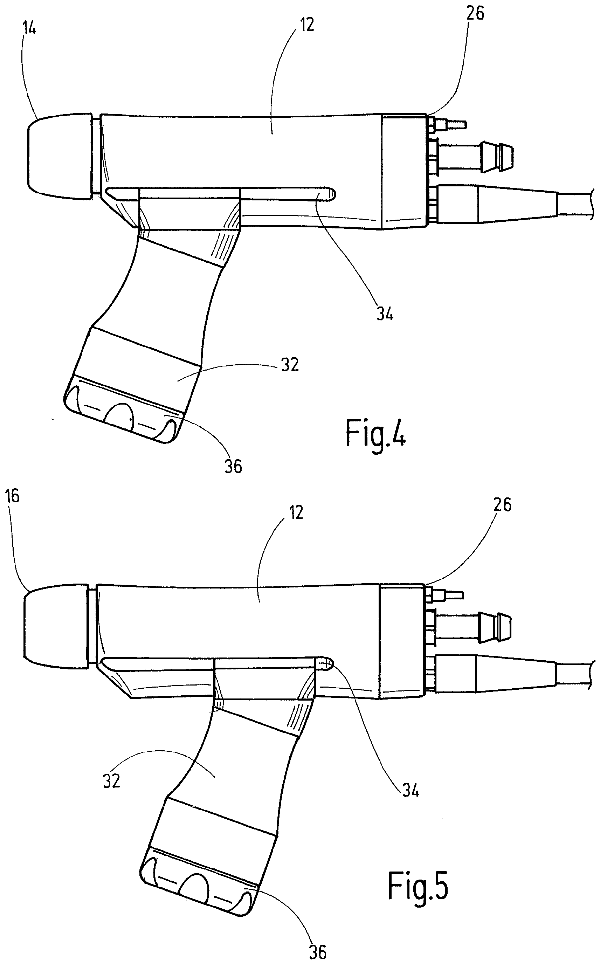

Die in Fig. 4 dargestellte Konfiguration unterscheidet sich dadurch von der in Fig. 2 dargestellten Konfiguration, dass der Handgriff 32 nicht mehr in Richtung des proximalen Endes 26 des Körpers 12 abgewinkelt ist, sondern dass dieser nun in Richtung des distalen Endes 14 des Körpers 12 abgewinkelt ist. Eine solche Stellung ist z.B. dann sinnvoll, wenn das medizinische Instrument nach unten geneigt werden soll. Um in diese Stellung zu kommen, wird wiederum durch Drehen des Drehknopfes 36 die Verriegelung des Handgriffs 32 gelöst und dieser durch Bewegen des Handgriffs 32 in Richtung des distalen Endes 14 des Körpers 12 aus den Nuten abgezogen. Der Handgriff wird dann um 180° gedreht und wieder in die Nuten 34 des Körpers 12 eingeführt und in Richtung des proximalen Endes 26 des Körpers 12 bewegt, bis die gewünschte Stellung erreicht ist. Durch Drehen des Drehknopfes 36 in umgekehrte Richtung wird der Handgriff 32 dann wieder an dem Körper 12 verriegelt.

Die in Fig. 5 dargestellte Konfiguration unterscheidet sich dadurch von der in Fig. 4 dargestellten Konfiguration, dass der Handgriff 32 wiederum weiter in Richtung des proximalen Endes 26 des Körpers 12 verschoben wurde. Um in diese Konfiguration zu gelangen, wird wiederum durch Drehen des Drehknopfes 36 die Verriegelung des Handgriffes 32 an dem Körper 12 gelöst und der Handgriff 32 wird in den Nuten 34 des Körpers 12 in Richtung des proximalen Endes 26 des Körpers 12 bewegt. Hat der Handgriff 32 die gewünschte Position erreicht, wird durch Drehen des Drehknopfes 36 in die andere Richtung wiederum der Handgriff 32 an dem Körper 12 verriegelt.

In Fig. 6 ist nun ein Handgriff zur Verwendung mit einem medizinischen Instrument zum Abtragen von Gewebe in Alleinstellung dargestellt. Dieser Handgriff entspricht im Wesentlichen dem in den Figuren 1 und 5 in Zusammenhang mit dem medizinischen Instrument 10 dargestellten Handgriff, so dass für die bereits beschriebenen gleichen Bauteile auch die gleichen Bezugszeichen verwendet werden.

In Fig. 6 ist ein Griff für ein medizinisches Instrument in seiner Gesamtheit mit der Bezugsziffer 32 bezeichnet. Der Handgriff 32 weist an seinem oberen Ende eine Ausnehmung 42 auf, die so ausgestaltet ist, dass diese das oder die medizinische(n) Instrument(e), mit dem (denen) der Handgriff verwendet werden soll, aufnehmen kann.

Im oberen Bereich der Ausnehmung 42 sind dabei zwei gegenüberliegende und im Wesentlichen parallele Schienen 44 angeordnet. Im vorliegenden Fall sind die Schienen 44 am oberen Ende der Ausnehmung 42 angeordnet. Diese können aber auch durchaus an weiter abgesenkten Positionen in der Ausnehmung 42 angeordnet sein, solange dadurch die Verbindung zwischen dem medizinischen Instrument und dem Handgriff 32 nicht beeinträchtigt wird.

Ferner sichtbar in dieser Darstellung ist eine Spitze 45 eines Fingers 54, der hier in einer ausgefahrenen Stellung dargestellt ist, die in dem Fall, dass der Handgriff an einem medizinischen Instrument angeordnet ist, jedoch nicht erreicht wird, wie später noch beschrieben wird. Der Finger 54 dient dazu, beim Festlegen des Handgriffs am Instrument gegen den Körper 12 und diesen entsprechend gegen die Schienen 44 zu drücken, was zur Verriegelung des Handgriffs 32 an dem Instrument führt.

In Fig. 7 ist der in Fig. 6 dargestellte Handgriff 32 nun im Schnitt dargestellt. In dieser Darstellung wird sichtbar, dass der am unteren Ende des Handgriffs angeordnete Drehknopf 36 an seiner Innenseite mit einem Innengewinde 46 kraftschlüssig verbunden ist. Dieses Innengewinde 46 greift in das Außengewinde eines Verschiebebolzens 48 ein. Der Verschiebebolzen 48 weist ferner einen Führungsbolzen 50 auf. Wird nun der Drehknopf 36 gedreht, so kann der Verschiebebolzen 48 aufgrund dessen, dass der Führungsbolzen 50 geführt wird, dieser Drehbewegung nicht folgen und führt daher eine translatorische Bewegung entlang der Längsachse des Handgriffs aus. Der Verschiebebolzen 48 wirkt mit einer Feder 52 zusammen, die sich gegen den Finger 54 abstützt. Eine Hülse 51, die die Feder 52 umgibt, ist mit dem Finger 54 verbunden. Die Hülse 51 weist eine schlitzartige Ausnehmung 53 auf, in die der relativ zur Hülse 51 bewegliche Führungsbolzen 50 eingreift.

Wird nun der Handgriff 32 an einem Körper, beispielsweise dem Körper 12 des Instruments 10, montiert, befindet sich der Finger 54 in seiner in Fig. 7 dargestellten gegenüber der Ausnehmung 42 zurückversetzten Stellung. In dieser Stellung ist die Spitze 45 des Fingers 54 in einer Innenfläche 43 der Ausnehmung 42 versenkt. Aus dieser Stellung ist der Finger 54 in Richtung zum Innenraum der Ausnehmung 42 hin bewegbar. Nachdem die Schienen 44 in entsprechende Nuten des Körpers des Instruments, an dem der Handgriff 32 befestigt werden soll, eingesetzt sind, wird der Drehknopf 36 gedreht, bis der Finger 54 den Körper des Instruments berührt. Bei dieser Berührung übt der Finger 54 jedoch noch keine Kraft auf den Körper des Instruments aus. Nun wird der Drehknopf 36 in gleicher Richtung weitergedreht, wodurch der Führungsbolzen 50 sich in die schlitzartige Ausnehmung 53 der Hülse 51 relativ zu Letzterer hineinbewegt. Gleichzeitig wird dabei der Verschiebebolzen 48 gegen die Feder 52 bewegt, wodurch diese langsam gespannt wird. Da sich die Hülse 51 wegen ihrer festen Verbindung mit dem Finger 54 und weil der Finger 54 gegen den Körper des Instruments anliegt, nicht weiter in Richtung zum Körper des Instruments hin bewegen kann, bildet ein Ende 55 der Ausnehmung 53 einen Anschlag für den Führungsbolzen 50. Das Spannen der Feder 52 und damit die Erhöhung der Kraft des Fingers 54 auf den Körper des Instruments ist somit durch diesen Anschlag begrenzt. Nunmehr ist der Handgriff 32 am Körper des Instruments verriegelt.

Das Abnehmen des Handgriffs 32 von dem Instrument erfolgt entsprechend auf umgekehrte Weise, wobei lediglich der Drehknopf 36 in entgegen gesetzter Richtung gedreht werden muss, bis der Finger 54 mit dem Körper des Instruments außer Eingriff kommt, wonach der Handgriff 32 vom Instrument abgenommen werden kann.

## Patentansprüche

1. Medizinisches Instrument zum Abtragen von Gewebe, in Form eines Shavers, Bohrers oder Morcellators, mit einem in Rotation versetzbaren Werkzeug und einem motorischen Antrieb zum Antreiben des Werkzeugs, und mit einem langgestreckten Körper (12), wobei am Körper (12) Anschlüsse (28, 30) vorhanden sind, von denen ein Anschluss (28) ein Stromanschluss ist; und mit einem seitlich von diesem langgestreckten Körper (12) abstehenden Handgriff (32), der abnehmbar an dem langgestreckten Körper (12) befestigt ist, so dass das medizinische Instrument bei angebrachtem Handgriff (32) über diesen ergreifbar ist,
**dadurch gekennzeichnet, dass**
das medizinische Instrument bei abgenommenem Handgriff (32) über den langgestreckten Körper (12) in Art eines Stabes haltbar ist,
der motorische Antrieb in Form eines Elektromotors ausgebildet ist, der im langgestreckten Körper (12) angeordnet ist, und
der Handgriff (32) so ausgebildet ist, dass er sowohl in distaler Richtung als auch in proximaler Richtung abgewinkelt an dem langgestreckten Körper (12) anbringbar ist.

2. Medizinisches Instrument nach Anspruch 1, **dadurch gekennzeichnet, dass** der Handgriff (32) in verschiedenen Positionen entlang der Längsachse an dem Körper (12) festgelegt werden kann.

3. Medizinisches Instrument nach Anspruch 2, **dadurch gekennzeichnet, dass** der Handgriff (32) stufenlos in verschiedenen Positionen entlang der Längsachse des Körpers (12) festgelegt werden kann.

4. Medizinisches Instrument nach Anspruch 2 oder 3, **dadurch gekennzeichnet, dass** der Handgriff (32) eine Verriegelung aufweist, mit der er in einer Position entlang der Längsachse des Körpers (12) verriegelt werden kann.

5. Medizinisches Instrument nach einem der Ansprüche 1 bis 4, **dadurch gekennzeichnet, dass** der Handgriff (32) eine Ausnehmung (42) zur Aufnahme des Körpers (12) aufweist, wobei im oberen Bereich der Ausnehmung (42) zumindest zwei im Wesentlichen parallele, einander gegenüberliegende Schienen (44) vorgesehen sind, die in dem Zustand, in dem der Handgriff (32) mit dem medizinischen Instrument (10) verbunden ist, in entsprechende, an dem Körper (12) angeordnete Nuten (34) eingreifen.

6. Medizinisches Gerät nach Anspruch 5, **dadurch gekennzeichnet, dass** der Handgriff (32) ferner zumindest einen verstellbaren Finger (54) aufweist, der aus einer Stellung, in der eine Spitze (45) des Fingers (54) in einer Innenfläche der Ausnehmung (42) versenkt ist, gegen den Körper (12) verspannbar ist, um den Handgriff (32) am Körper (12) zu verriegeln.

7. Medizinisches Instrument nach einem der Ansprüche 1 bis 6, **dadurch gekennzeichnet, dass** der Handgriff (32) in einem Winkel von dem Körper (12) absteht der ungleich 90° ist.

## Claims

1. Medical instrument for removing tissue, in the form of a shaver, drill, or morcellator, comprising a tool that can be set in rotation and a motorized drive for driving the tool, and comprising an elongated body (12), wherein at the body (12) connectors (28, 30) are provided, one connector (28) of which is a power connector; and comprising a handle (32) that projects laterally from said elongated body (12), that is fastened in a detachable manner to the elongated body (12) so that the medical instrument can be grasped via the a handle (32) when the handle is attached, **characterized in that**
the medical instrument can be held like a rod via the elongated body (12) when the handle (32) is detached,
the motorized drive is arranged in the form of an electromotor that is positioned in the elongated body (12), and
the handle (32) is designed in such a way that it is attachable to the elongated body (12) both in a manner inclined in the distal direction and in the proximal direction.

2. Medical instrument according to Claim 1, **characterized in that** the handle (32) can be fixed to the body (12) at different positions along the longitudinal axis.

3. Medical instrument according to Claim 2, **characterized in that** the handle (32) can be fixed in a stepless manner at different positions along the longitudinal axis of the body (12).

4. Medical instrument according to Claim 2 or 3, **characterized in that** the handle (32) comprises a locking means, by way of which it can be locked in a position along the longitudinal axis of the body (12).

5. Medical instrument according to one of Claims 1 to 4, **characterized in that** the handle (32) comprises a recess (42) for accommodating the body (12), wherein at least two substantially parallel bars (44) which are located opposite one another are provided in the upper region of the recess (42) and, in the state in which the handle (32) is connected to the medical instrument (10), engage corresponding grooves (34) that are arranged on the body (12).

6. Medical instrument according to Claim 5, **characterized in that** the handle (32) further comprises at least one adjustable finger (54), which, from a position in which a tip (45) of the finger (54) is recessed in an inner surface of the recess (42), can be biased against the body (12) in order to lock the handle (32) to the body (12).

7. Medical instrument according to one of Claims 1 to 6, **characterized in that** the handle (32) projects from the body (12) at an angle other than 90°.

## Revendications

1. Instrument médical pour l'ablation de tissus, sous la forme d'un rasoir, d'une mèche ou d'un morcellateur, comprenant un outil pouvant être mis en rotation et un entraînement à moteur pour l'entraînement de l'outil, et comprenant un corps allongé (12), des raccords (28, 30) étant prévus sur le corps (12), dont un raccord (28) est un raccord électrique ; et comprenant une poignée (32) partant latéralement depuis ce corps allongé (12), qui est fixée de manière amovible au corps allongé (12), de telle sorte que l'instrument médical puisse être saisi par la poignée lorsque celle-ci est montée (32),
**caractérisé en ce que**
l'instrument médical, lorsque la poignée est enlevée (32), peut être tenu par le biais du corps allongé (12) sous la forme d'une barre,
l'entraînement à moteur est réalisé sous forme de moteur électrique qui est disposé dans le corps allongé (12), et
la poignée (32) est réalisée de telle sorte qu'elle puisse être montée sur le corps allongé (12) sous forme inclinée dans la direction distale ainsi que dans la direction proximale.

2. Instrument médical selon la revendication 1, **caractérisé en ce que** la poignée (32) peut être fixée sur le corps (12) dans différentes positions le long de l'axe longitudinal.

3. Instrument médical selon la revendication 2, **caractérisé en ce que** la poignée (32) peut être fixée le long de l'axe longitudinal du corps (12) dans diverses positions de manière continue.

4. Instrument médical selon la revendication 2 ou 3, **caractérisé en ce que** la poignée (32) présente un verrouillage avec lequel elle peut être verrouillée dans une position le long de l'axe longitudinal du corps (12).

5. Instrument médical selon l'une quelconque des revendications 1 à 4, **caractérisé en ce que** la poignée (32) présente un évidement (42) pour recevoir le corps (12), au moins deux rails (44) mutuellement opposés, essentiellement parallèles, étant prévus dans la région supérieure de l'évidement (42), lesquels viennent en prise dans des rainures correspondantes (34) disposées sur le corps (12) dans l'état dans lequel la poignée (32) est connectée à l'instrument médical (10).

6. Instrument médical selon la revendication 5, **caractérisé en ce que** la poignée (32) présente en outre au moins un doigt réglable (54) qui peut être serré contre le corps (12) depuis une position dans laquelle une pointe (45) du doigt (54) est enfoncée dans une surface intérieure de l'évidement (42), afin de verrouiller la poignée (32) sur le corps (12).

7. Instrument médical selon l'une quelconque des revendications 1 à 6, **caractérisé en ce que** la poignée (32) fait saillie du corps (12) suivant un angle différent de 90°.
